# EUROPEAN PATENT SPECIFICATION

(11) **EP 2 254 464 B1**
(45) Date of publication and mention of the grant of the patent: **04.01.2017**
(21) Application number: 09712414.3
(22) Date of filing: 11.02.2009
(51) Int. Cl.: A61B 5/00, A61B 5/07, A61M 31/00, A61B 5/145

(54) **ADMINISTRATION OF DRUGS TO A PATIENT**
VERABREICHUNG VON WIRKSTOFFEN AN EINEN PATIENTEN
ADMINISTRATION DE MÉDICAMENTS À UN PATIENT

(30) Priority: 18.02.2008 US 29447
(43) Date of publication of application: 01.12.2010
(73) Proprietor: Medimetrics Personalized Drug Delivery B.V., 5656 AE, Eindhoven (NL)
(72) Inventor: SHIMIZU, Jeff, NL-5656 AE Eindhoven (NL)
(74) Representative: Flügel Preissner Schober Seidel
(86) International application number: PCT/IB2009/050553
(87) International publication number: WO 2009/104110

(56) References cited:
- WO-A-2006/021932
- WO-A-2006/056944
- WO-A-2007/072297
- WO-A-2008/099356
- WO-A2-2004/066903
- US-A1- 2005 147 559
- US-A1- 2006 145 876

## Description

### FIELD OF THE INVENTION

The invention relates to an electronic pill for the administration of at least one drug to a patient.

### BACKGROUND OF THE INVENTION

The WO 2005/038049 A2 describes the adjustment of drug formulations including beads, pellets, granules, coatings etc. to the individual drug-related parameters of a patient. The disclosed method requires different preparations for each patient, which is very time consuming and cost intensive.

US 2005/147559 A1, WO 2006/056944 A1, WO 2007/02297 A2, WO 2006/021932 A1, US 2006/0145876 A1, WO 2008/099356 A2 and WO 2004/066903 A2 disclose using an electronic pill for the administration of a drug.

### SUMMARY OF THE INVENTION

Based on this situation it was an object of the present invention to provide means for a more efficient and/or safe drug administration to a patient. In particular, it is desirable to create and maintain a therapeutic concentration of a drug in systemic circulation.

This object is achieved by an electronic pill according to claim 1. Preferred embodiments are disclosed in the dependent claims.

The invention relates to an electronic pill for the administration of at least one drug to a patient, said electronic pill comprising a drug reservoir and a controller for the controlled delivery of the drug from said drug reservoir with a delivery profile that is determined according to at least one individual parameter of the patient, wherein the individual parameter of the patient comprises: a part of the genotype of the patient wherein the part of the genotype of the patient relates to the cytochrome expression and the cytochrome expression relates to a cytochrome P-450 enzyme.

The aforementioned electronic pill provides the hardware with which an exemplary method of the kind described in the following can be executed. Reference is therefore made to the following description of the method for more information about the electronic pill, its advantages and modifications. It should be noted that the electronic pill may particularly comprise a memory for storing a computer program of the kind described the following.

The exemplary method associated with the electronic pill according to the present invention serves for the administration of at least one drug to a patient, wherein the terms "drug" and "patient" are to be understood in the most general sense, i.e. referring to any kind of pure or composed substance that shall be administered to a (sick or healthy) human or animal body, respectively. The method comprises the delivery of the at least one drug by an electronic pill with a delivery profile that is determined according to at least one individual parameter of the patient. As usual, the term "electronic pill" shall denote a capsule with some electronics that can be ingested by the patient and that controllably emits the drug from a reservoir during its passage through the gastrointestinal tract. Electronic pills are for example described in the documents US 2007 0213659 A1, WO 2006/025013 A1, and WO 2007/148238 A1.

The described method provides an administration of a drug according to a profile that is individually tailored to the requirements of a patient. This can dramatically improve the effectiveness of the drug and also increase the safety of the treatment as overdoses of the drug can be avoided. The method allows creating and maintaining a therapeutic concentration of a drug in systemic circulation in spite of the fact that the therapeutic window may be narrow and the bioavailability can be highly variable between patients. To achieve these positive effects, the method requires only a corresponding programming of the electronic pill, which is not more complicated than the programming with a standard profile. Moreover, the electronic pill can realize the individual delivery profile with much higher precision than for example particular formulations (pellets, coatings etc.) of the drug.

The individual parameter according to which the drug delivery profile is set may comprise any quantity or value that has some relation to the drug and its effects and that may vary from patient to patient or (for one patient) from time to time. The individual parameter comprises is part of the genotype of the patient, for instance in regard to cytochrome expression as the cytochrome P-450 enzymes are important factors in the elimination of commonly used drugs. The individual parameter may further comprise a part of the phenotype of the patient, for instance in regard to the expression of particular biomolecules in the gastrointestinal tract that are known to affect the absorption/rejection of a drug. The individual parameter may as an example further be related to a particular substance in the body of the patient that is correlated to the administered drug, for instance a metabolic product in the blood, urine or breath of the patient; the occurrence and/or amount of this substance may then provide valuable information about desired or undesired effects of the drug. Another example of individual parameters are pharmacokinetic data and/or the response of the patient to parameter may comprise additional information like the profile of co-administered drugs which may interact with the drug to be administered by the electronic pill.

A particularly important set of individual parameters (which may overlap with the aforementioned ones) relates to the expected response of the patient to the drug administration, wherein this response may for example relate to the metabolism of the administered drug and/or its absorption or efflux. The expected response of the patient may be estimated from preliminary physiological measurements. Taking it into account helps to fit the drug delivery profile to an individual patient right from the beginning of a therapy.

In another aspect, the individual parameter relates to the (spatial or local) distribution of particular proteins (e.g. receptors) in the gastrointestinal tract of the patient, particularly the distribution of members of the ATP-binding cassette family like P-glycoprotein or multidrug resistance protein. Thus the delivery profile can be adapted to the locally varying conditions in the gastrointestinal tract of a patient and particularly be adjusted to a target region of optimal absorption conditions.

The applied delivery profile is usually characterized by one or more adjustable parameters, for example by the total amount of drug that shall be administered, by the composition of the applied drug-cocktail (comprising e.g. the co-administration of bioenhancers), or by the consistency of the drug (powder, liquid, etc.). The drug profile may especially be characterized by the drug dispense rate (e.g. measured in µg of drug expelled by the electronic pill per minute) versus time and/or versus location in the gastrointestinal tract. A drug dispense rate that is given as a function of time is usually based on an average passage of the electronic pill through the gastrointestinal tract of the patient. An optimized direction of the drug delivery to particular target regions can be achieved if the drug dispense rate depends on the location in the gastrointestinal tract. Appropriate means for making the dispense rate dependent on the location in the gastrointestinal tract are for example described in literature (e.g. D.F. Evans et al, Gut 1988, 29, 1035-1041; UB Kompella and VHL Lee, "Delivery systems for penetration enhancement of peptide and protein drugs: design considerations", Advanced Drug Delivery Reviews, Vol. 46, pp. 211-245, 2001).

During the administration of the drug, pharmacokinetic data and/or a patient response may be sampled. The sampling may for example be done by external devices like an electrocardiographic recorder, or it may be based on the repeated taking of samples from blood, urine, or breath. In particular, the sampling may be done by the electronic pill itself if this is equipped with appropriate sensors. The sampled data may for instance be used to evaluate the effect of the drug administration.

It is possible to determine an individual delivery profile for a patient once and to apply this continuously during one or more administrations of the electronic pill. In a preferred example, the delivery profile of the drug is however adjusted during and/or after the administration of the drug, thus allowing a continuous improvement of the drug administration in a feedback loop. The delivery profile may particularly be adjusted during the administration of the drug according to one or more values measured by the electronic pill, for example according to the pH value in the gastrointestinal tract or according to the sensed cytochrome activity.

The exemplary method will typically be realized with the help of a computing device, e.g. a workstation, where data is gathered and the desired delivery profile is determined. Data parameters for the profile are then downloaded to the electronic pill, which is programmable and may include as a computing device a microprocessor or an FPGA. Accordingly, an example further includes a computer program product which provides the functionality of any of the methods when executed on a computing device.

Further, the present description describes a data carrier, for example a floppy disk, a hard disk, an EPROM, or a compact disc (CD-ROM), which stores the computer product in a machine readable form and which executes at least one of the methods when the program stored on the data carrier is executed on a computing device. The data carrier may particularly be suited for storing the program of the computing device (e.g. workstation, microprocessor) mentioned in the previous paragraph. The pill program will typically reside in a microprocessor of the electronic pill and is likely to be loaded at the time of manufacturing.

Nowadays, such software is often offered on the Internet or a company Intranet for download, hence the present invention also includes transmitting the computer product over a local or wide area network. The computing device may include one of a workstation, a microprocessor and an FPGA.

### BRIEF DESCRIPTION OF THE DRAWINGS

These and other aspects of the invention will be apparent from and elucidated with reference to the embodiment(s) described hereinafter. These embodiments will be described by way of example with the help of the accompanying drawings in which:
Figure 1 shows schematically the components of an electronic pill system;
Figure 2 shows a section through an exemplary electronic pill:
Figure 3 illustrates an exemplary method of drug administration.

Like reference numbers in the Figures refer to identical or similar components.

### DETAILED DESCRIPTION OF EMBODIMENTS

Problems with limited oral bioavailability are present with a wide variety of drugs. In general the oral route is always preferred when feasible. However if the drug fails to enter systemic circulation after oral administration (poor bioavailability) then it must be delivered by an alternate route. Often this is by injection. Delivery by injection can bring significant additional burdens including need for professional administration, pain and discomfort, sterilization requirements, and injection reactions.

For various reasons many orally delivered compounds exhibit good uptake in relatively localized regions in the intestines. There are many examples of drugs with site-specific absorption. Factors contributing to the differences include gut composition, mucus thickness, pH, surface area; and enzyme activity. Among the non-peptide drugs known to exhibit regional selective absorption are ciprofloxacin, piretanide, metoprolol, and baclofen (cf. Siccardi D., Adv. Drug Deliv. Rev., 57(2): 219-35 (2005)). Current drug targeting approaches generally rely on enteric coatings to protect a matrix of the drug. After the coating dissolves the drug matrix dissolves thus releasing the drug for absorption through the gut wall. The dissolution process is variable and results are often imperfect.

Moreover, differences in drug response among patients are common. Therapies are developed to provide benefit to the "average" patient while minimizing adverse reactions. One known variable is the patient's profile of the cytochrome P-450 enzymes, an important contributor to oxidative metabolism. These enzymes account for almost 50 percent of.the overall elimination of commonly used drugs. There are many enzymes in the P-450 family classified by their amino acid similarities. The enzyme activity can be affected by diet, concurrent medications, or inherited variations. The wide range in activity can markedly impact response to a drug and risk for adverse reaction. For example an individual with low activity will slowly metabolize the drug and it can accumulate into toxic concentrations over the course of treatment.

The liver is the major site of cytochrome P-450 metabolism. However the intestines are also an important site, especially for CYP3A. CYP3A metabolizes a wide array of clinically important drugs and is the most abundant form in the intestines. In a study of twenty human donors, CYP3A represented 63%, 49% and 88% of total small intestinal cytochromes in the duodenum, jejunum, and ileum, respectively (Paine M.F., et. al., J. Pharmacol. Exp. Ther., 283(3):1552-62 (1997)). There is a progressive decline in CYP3A content from the duodenum to jejunum to ileum. Thus the upper small intestines represent the major site for intestinal CYP-mediated first past metabolism. Results also show significant interindividual variability and this can account for the large differences in oral bioavailability observed in some CYP3A drug substrates. Duodenal and jejunal CYP3A content varied by more than 30-fold among the tewnty donors in the study.

The oral bioavailability of many cytotoxic cancer drugs is low and/or highly variable. Typically they are administered by intravenous injection once every few weeks. In addition to the cost, convenience and compliance advantages that would come from an oral formulation, there is evidence that a more frequent dosing schedule facilitated by oral administration would improve outcomes. In vitro, increasing the duration of taxane exposure above a threshold level is more important than achieving high peak concentrations (Engels F.K., et. al., Br. J. Cancer, 93(2):173-7 (2005)). Taxanes include placlitaxel and docetaxel, widely used in ovarian, breast, and some lung cancers.

Factors responsible for the low or variable bioavailability of cytotoxic drugs include high affinity for drug transporters and activity of metabolic enzymes. Many tissues that form a protective barrier, including the epithelial membrane of the intestines, include an excretory function for transporting substances back out of the cell. These drug transporters include P-glycoprotein (P-gp) and multidrug resistance protein (MRP) and are members of the ATP-binding cassette family (ABC). These transporters can also confer multidrug resistance to cancer cells. There is extensive first pass metabolism of taxanes and other cancer drugs by CYP3A. Further an overlap in selectivity and localization of CYP3A and P-gp suggest these two proteins cooperate and constitute an absorption barrier.

Recognizing that these efflux pumps and CYP metabolism can limit the bioavailability of many drug candidates, strategies have been proposed to inhibit these mechanisms (e.g. US 5 567 592, US 6 803 373, US 7 030 132).
In targeting regions in the intestinal tract it is possible to formulate the drug by chemical means using enteric coating, degradable matrices, beads, and the like. Performance of the delivery vehicle is then however based on averages and can vary significantly. Further the range in profiles achievable and the ability to target narrow regions is limited.

Generally there is a single drug formulation for a given therapy. This formulation will target a given location with a given target release rate. Thus it is a one size fits all approach based on the average best fit while minimizing adverse reactions. As discussed above, the absorption profile can however vary significantly between individuals. The ability to deliver a drug of limited and variable bioavailability may thus be dependent on the ability to tailor the delivery profile to the individual.

To address the above issues, the present invention proposes to use an electronic pill system in a strategy to deliver a drug to the appropriate areas of the intestinal tract and in concentrations tailored to the individual. In this manner bioavailability is improved and variation between individuals is reduced. This can enable the oral delivery of drugs that must presently be injected due to bioavailability problems.

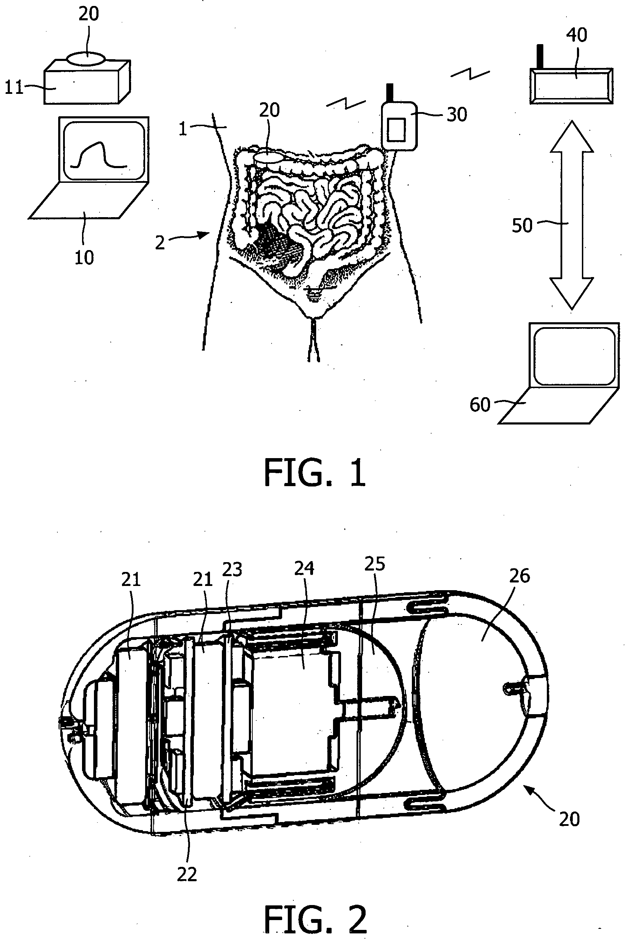

An exemplary electronic pill 20 ("ePill") is illustrated in Figure 2. The pill itself is one component of a larger "ePill system", as shown in Figure 1. This system includes the following components:
- a programming station 10, 11 for programming a given drug delivery profile into ePills 20;
- a set of ingestible ePills 20;
- a portable unit 30 for wirelessly gathering data from a pill 20 during its passage through the gastrointestinal tract 2 of a patient 1;
- a home base station 40 to gather data from the portable unit 30;
- a network 50 for transferring the pill data to the computer 60 of an interested party such as the doctor.

In the embodiment of Figure 2, the ePill 20 used for drug delivery has the following elements: a battery 21, PICS (power integrated circuits) 22 for microprocessor and receiver, a flex foil 23 with bumped electronic components, a miniature electrical motor 24, a piston 25, a drug reservoir 26, and sensors.

The invention uses an electronic pill of the kind described above. The delivery profile of the pill may be easily programmed before ingestion by the patient. Further the delivery profile may be tailored to the individual. The drug reservoir may be loaded with the target compound that normally has limited and variable bioavailability. The delivery profile may be determined by the input of one or more data sources. The data sources may be the individual's genetic profile (e.g. determined by the AmpliChip CYP450 microarray of Roche Diagnostics), measurement of expression transport receptors or presence of enzymes in the gut, measurements of the amount of drug delivered into circulation in the individual, or individual response to treatment. The electronic pill may be controlled to release the drug at a specified rate and time. This results in delivery of the drug to target location(s) in the gastrointestinal (GI) tract at a target concentration level(s).

The drug contained in the reservoir 26 is typically one that suffers from poor and/or highly variable oral bioavailability. The reasons for the poor bioavailability profile can be many. At a minimum the bioavailability of the drug can be improved by delivering to a relatively small region of the GI tract. A first step in the method of delivery is therefore to determine the target location of delivery. This may be known for a given drug by previous experimentation. Next the pill and system are programmed to accurately target that area. The pill is then ingested by the patient and the actions of the pill as controlled by the program will deliver the drug to the area of improved absorption, thus improving bioavailability.

There are many drugs that show good or improved absorption in only a limited region. For example consider salmon calcitonin. A study of regional absorption in rats determined the location of maximum absorption is in the ileum. The drug reservoir of the pill may be loaded with a formulation of salmon calcitonin and the pill may then be programmed to release drug only in the ileum. Location targeting may be achieved by several strategies. For example, the pill may contain a pH sensor. The pH sensor can reliably determine when the pill exits the stomach by a tell-tale rise in pH. Transit of the pill in the human small bowel is reasonably repeatable. Thus the pill could wait a pre-determined amount of time and begin to release contents over a second time interval. The delay time and release time coincide with norms of GI transit so as to target release in the ileum, for this example.

Another example of an area where the proposed approach can improve bioavailability is in the delivery of large molecules such as proteins or peptides. Oral delivery of protein and peptides is a topic of extensive research and development. The compounds are degraded by the harsh environment of the stomach, enzymes of the upper GI tract, and are poorly absorbed through the epithelium. This has led to research in a number of strategies for protecting the compound and promoting uptake. Among the many approaches are mucoadhesive systems, receptor mediated transport, location targeting, liposomes, polymer shells, complex formation with a carrier, and temporary opening of tight junctions. The properties of ePill can be quite beneficial to drug delivery strategies in this case. First the compounds are well protected in the drug reservoir from the GI environment until they are dispensed at the target location. Next, ePill can accurately target the location for delivery where the compound is best absorbed. Location targeting and accurate control of the release profile ensures repeatable delivery both in place and concentration. It is probable that the ePill strategy can also make use of drug delivery developments that aim to improve uptake of the compound across the gut wall. That is formulations that make use of carrier or promoter compounds can be made and stably loaded into the drug reservoir or multiple reservoirs if needed.

In typical embodiments, the drug not only exhibits a regional preference for delivery but also shows potentially large bioavailability variation between individuals. Thus the method should also include an early step to characterize the expected response of the individual. This characterization is used to program the pill and system with a release profile tailored to that person. After programming and administration of the pill, the method may also include a step to evaluate the response of the individual. This response is then used to tailor the release profile of subsequent administration of the pill.

For example, the drug may be a substrate for cytochrome P-450 metabolism and/or subject to efflux back into the intestinal lumen. The drug may be recognized by P-glycoprotein, BCRP, or other systems responsible for removal of drugs from the intestinal enterocytes and forming a barrier to absorption. In this case bioavailability is improved by the co-administration of a bioenhancer that serves to inhibit CYP metabolism and/or drug efflux. Several examples have been cited where a bioenhancer improves bioavailability thus opening up possibility of oral administration. As a typical example, the delivery of taxane compounds for chemotherapy may be considered. Effective bioenhancement is possible with co-administration of cyclosporine and with second generation enhancers such as elacridar.

In an exemplary method first there is an evaluation of the patient profile. This may include a genetic test to characterize the individual's genotype in regard to cytochrome expression. For example the AmpliChip CYP450 from Roche Diagnostics can be used for this case. Differences in CYP expression can dramatically affect the bioavailability of the drug and also the effectiveness of the bioenhancer intended for CYP inhibition. Results will guide the programming of the ePill system and administration method with regard to dosing levels of both the drug and the bioenhancer. Preferably, but not necessary, there is also a step to characterize the concentration of CYP, P-gp, or BCRP along the intestinal tract. This might be accomplished by means of a diagnostic test including application of a dye-tagged anti-body against for example P-gp. Measurement of fluorescent intensity can then be accomplished by an endoscope procedure, preferably by a capsule endoscope procedure that includes fluorescent imaging capability. Alternatively this could be accomplished by a diagnostic test using a probe drug such as digoxin and midazolam and measuring blood and urine samples (Kirby B., et. al., J. Clin. Pharmacol., 46(11):1313-9 (2006)). Less invasive characterization could be accomplished with a tagged erythromycin breath and urine test (Lemahieu W.P., et. al. Am. J. Physiol. Gastrointest. Liver Physiol., 285(3):G470-82 (2003)).

Data of the patient's CYP genotype and local concentration of P-gp activity may then be used to program the delivery profile of the ePill. The ePill is loaded with the target formulation and the delivery profile, concentration versus location, is programmed into the pill. The target formulation may for example be paclitaxel. The formulation may include also the presence of the bioenhancer, for example elacridar. Alternatively the bioenhancer may be administered orally in a separate pill taken before or concurrently with the ePill.

After ingestion of the pill the individual response to the treatment may be measured. For example blood tests may determine the actual amount of target drug, for example paclitaxel, that enters systemic circulation. This can be particularly important when the drug has significant side effects, poorly predictable bioavailability, or a narrow therapeutic window. After measuring the actual bioavailability of the target drug for that person, the release profile for future use of the ePill can be tailored to achieve ideal concentrations. Over the course of treatment the patient response at a disease or other marker level could be measured and again the dosage profile of future pills adapted accordingly.

In an alternate example the ePill itself may contain a sensor for CYP activity. In this case during the course of pill transit the real time activity level is determined and adjustments in drug delivery rate can be made en-route. The sensor could for example employ a degradeable polymer sensitive to the cytochrome enzyme or some surrogate compound.

In summary the exemplary method preferably employs the following steps:
- characterize the expected patient response (metabolism, efflux) to the drug treatment,
- map the expected absorption profile in the patient's intestinal tract for example by concentration of enzymatic activity or expression of relevant receptors,
- create a drug delivery profile of dispense rate versus location,
- administer the ePill,
- gather pharmacokinetic data or monitor response,
- adjust delivery profile, and repeat as necessary from administer the ePill.

The pill may be used for oral administration of a therapeutic drug, particularly a drug that suffers from poor or variable bioavailability. They allow to create and maintain a therapeutic concentration of the drug in systemic circulation by measuring some parameter(s) and adjusting the dispense profile for dose and target location. Bioavailability is improved by location targeting the delivery of the drug by use of a programmable electronic pill system. Use of a bioenhancer may also be included. This allows oral administration of drugs that would otherwise require delivery by injection.

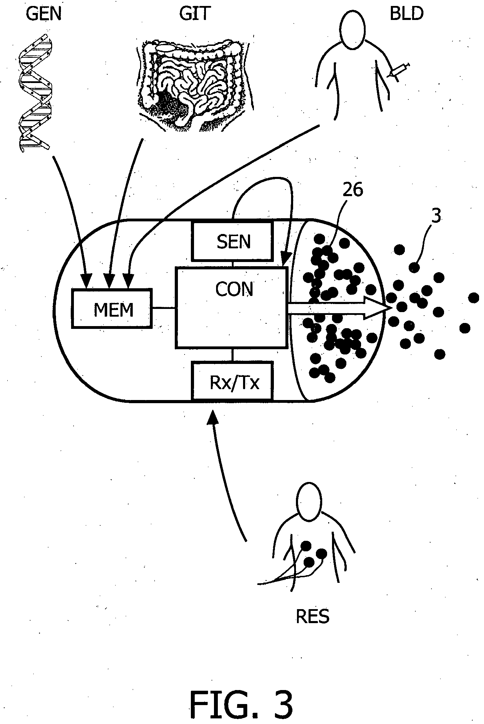

Figure 3 illustrates in a diagram different example of the drug administration method described above. The schematically drawn capsule of an electronic pill 20 comprises inter alia the following components (e.g. as hardware and/or software modules):
- A programmable memory MEM, e.g. realized by a flash memory or RAM embedded in the microprocessor.
- A controller CON, e.g. realized by a microcontroller, which controls (inter alia) the delivery of the drug 3 from the reservoir 26.
- At least one sensor SEN, e.g. for CYP or pH.
- A receiver/transmitter Rx/Tx for a wireless communication with external devices.

The delivery program of the drug that is executed by the controller CON is based on data stored in the memory MEM, on feedback from the internal sensor SEN, and/or on feedback from external measurements about the patient response RES. The data stored in advance in the memory MEM may be based on various data sources, for example:
- The genotype GEN of the patient.
- The specification of target regions in the gastrointestinal tract GIT of the patient, thus allowing an intestinal targeting.
- Pharmacokinetic data BLD including the response of the patient during previous administrations of the drug.

Finally it is pointed out that in the present application the term "comprising" does not exclude other elements or steps, that "a" or "an" does not exclude a plurality, and that a single processor or other unit may fulfill the functions of several means. The invention resides in each and every novel characteristic feature and each and every combination of characteristic features. Moreover, reference signs in the claims shall not be construed as limiting their scope.

## Claims

1. An electronic pill (20) for the administration of at least one drug (3) to a patient (1), comprising a drug reservoir (26) and a controller (22, 23, CON) for the controlled delivery of the drug from the drug reservoir with a delivery profile that is determined according to at least one individual parameter of the patient, wherein the individual parameter of the patient comprises the following parameter:
a part of the genotype of the patient wherein the part of the genotype of the patient (1) relates to a cytochrome expression and **characterized in that** the cytochrome expression relates to a cytochrome P-450 enzyme.

2. The electronic pill according to claim 1, wherein the individual parameter of the patient comprises the phenotype of the patient (1), wherein the part of the phenotype of the patient (1) relates to an expression of proteins in the gastrointestinal tract of the patient.

3. The electronic pill according to claim 1, **characterized in that** the pill includes a sensor for detecting the cytochrome expression.

4. The electronic pill according to claim 2, **characterized in that** the expression of proteins relates to P-glycoprotein and multidrug resistance protein (MRP).

## Patentansprüche

1. Elektronische Pille (20) zum Verabreichen zumindest eines Medikaments (3) an einen Patienten (1), umfassend einen Medikamentenbehälter (26) und eine Steuerung (22, 23, CON) zur kontrollierten Abgabe des Medikaments aus dem Medikamentenbehälter mit einem Abgabeprofil, das nach zumindest einem individuellen Parameter des Patienten festgelegt wird, wobei der individuelle Parameter des Patienten den folgenden Parameter umfasst:
einen Teil des Genotyps des Patienten, wobei der Teil des Genotyps des Patienten (1) sich auf eine Cytochrom-Expression bezieht, **dadurch gekennzeichnet, dass** sich die Cytochrom-Expression auf ein Cytochrom-P450-Enzym bezieht.

2. Elektronische Pille nach Anspruch 1, wobei der individuelle Parameter des Patienten den Phänotyp des Patienten (1) umfasst, wobei der Teil des Phänotyps des Patienten (1) sich auf eine Expression von Proteinen im Gastrointestinaltrakt des Patienten bezieht.

3. Elektronische Pille nach Anspruch 1, **dadurch gekennzeichnet, dass** die Pille einen Sensor zum Detektieren der Cytochrom-Expression beinhaltet.

4. Elektronische Pille nach Anspruch 2, **dadurch gekennzeichnet, dass** die Expression von Proteinen sich auf P-Glyokprotein und Multidrug-Resistance-Protein (MRP) bezieht.

## Revendications

1. Pilule électronique (20) pour l'administration d'au moins un médicament (3) à un patient (1), comprenant un réservoir de médicament (26) et un contrôleur (22, 23, CON) pour la distribution contrôlée du médicament depuis le réservoir de médicament avec un profil de distribution qui est déterminé en accord avec au moins un paramètre individuel du patient, dans laquelle le paramètre individuel du patient comprend le paramètre suivant :
une partie du génotype du patient, tel que ladite partie du génotype du patient (1) est en relation avec une expression cytochrome, et
**caractérisée en ce que** l'expression cytochrome est en relation avec un enzyme cytochrome P-450.

2. Pilule électronique selon la revendication 1, dans laquelle le paramètre individuel du patient comprend le phénotype du patient (1), dans laquelle la partie du phénotype du patient (1) est en relation avec une expression de protéines dans le système gastro-intestinal du patient.

3. Pilule électronique selon la revendication 1, **caractérisée en ce que** la pilule inclut un capteur pour détecter l'expression cytochrome.

4. Pilule électronique selon la revendication 2, **caractérisée en ce que** l'expression de protéines est en relation avec la P-glycoprotéine et une protéine de résistance à une multiplicité de médicaments (MRP).
